# EUROPEAN PATENT APPLICATION

(11) **EP 4 711 445 A1**
(43) Date of publication of application: **18.03.2026**
(21) Application number: 24807171.4
(22) Date of filing: 10.05.2024
(51) Int. Cl.: C12N 5/07, C07K 1/16, C07K 1/30, G01N 1/10, G01N 15/04, G01N 30/06, G01N 30/46, G01N 30/80, G01N 30/88

(54) **METHOD FOR SEPARATING AND FRACTIONATING BIOLOGICAL SAMPLE**

(30) Priority: 12.05.2023 JP 2023079120
(71) Applicant: Early Reflections Co., Ltd., Tokyo 101-0051 (JP)
(72) Inventor: OGAWA, Tadayuki, Shimotsuga-gun, Tochigi 321-0293 (JP)
(74) Representative: Schott, Jakob Valentin
(86) International application number: PCT/JP2024/017546
(87) International publication number: WO 2024/237219

(57) **Abstract**

This method for separating and fractionating a biological sample includes: a homogenization step for preparing a cell homogenate; a first centrifugation step for separating the cell homogenate into a first supernatant component and a first pellet component; a stepwise centrifugation step for separating an (m-1)-th supernatant component into an m-th supernatant and an m-th pellet component, wherein m is an integer from 2 to n; a density gradient centrifugation step for subjecting a density gradient sample, which is prepared by adding the m-th pellet component to a density gradient reagent, to density gradient centrifugation; a density gradient centrifugal recovery step for fractionating the density gradient sample in a density gradient centrifuge tube into aₘ fractions in order from the top or bottom side; and a chromatography step for fractionating an n-th supernatant component into b fractions by size exclusion chromatography.

## Description

### Technical Field

The present invention relates to a method for separating and fractionating, with high resolution, substances and structures that constitute a biological sample collected from a human body or the like.

### Background Art

The body of a human, an animal, or the like is an orderly assembly of various substances. Substances constituting a living body exhibit appropriate molecular functions within the living body by maintaining appropriate order, such as localization at appropriate sites, and appropriate morphology and size. However, when such appropriate order is disturbed for some reason, the disturbance of the appropriate order leads to modulation of the substances, which manifests as a disease. For example, it is known that, as molecular-level pathological conditions in various diseases, phenomena such as changes in molecular localization and changes in molecular size, including the formation of dimers, multimers, and aggregates, occur, and aggregation of amyloid-related proteins in Alzheimer's disease and multimerization of pathological proteins in prion disease have been observed. Accordingly, in order to understand diseases, molecular-level analyses that can accurately evaluate variations in localization and size distribution of particles and molecules contained in biological samples are required.

Such analyses of biological molecules consist of a "separation and fractionation technique" for separating and fractionating molecules from biological tissues and a "detection technique". As the "separation and fractionation technique", centrifugation methods such as those described in NPL 1 have been used for a long time in order to extract and prepare large intracellular components from biological tissues. As improvements on methods in which the solution density and the centrifugal force are constant, stepwise centrifugation method in which the centrifugal force is changed stepwise (see NPL 2), density gradient centrifugation method in which a density gradient is used (see NPL 3), and the like are also known. Further, liquid chromatography is generally used for molecular groups of soluble components that do not precipitate even when ultracentrifugation method in which centrifugation method is performed at ultrahigh speed. On the other hand, regarding the "detection technique", instruments for detecting various substances such as proteomics, metabolomics, and metallomics have dramatically advanced, making it possible to perform various analyses and detections of molecules and elements, such as molecular identification, structural analysis, molecular modification analysis, and elemental composition analysis.

### Citation List

### Non-Patent Literature

NPL 1: Harvey R. Cloten et al., "Purification of the first component of complement by zonal ultracentrifugation", Journal of Immunology, 103(4), 862-865, 1969
NPL 2: Aikaterini Geladaki et al., "Combining LOPIT with differential ultracentrifugation for high-resolution spatial proteomics", Nature Communications, 10(1), 331 2019
NPL 3: T. Ford et al., "Iodixanol: a nonionic iso-osmotic centrifugation medium for the formation of self-generated gradients", Analytical Biochemistry, 220(2), 360-366, 1994

### Summary of the Invention

### Technical Problem

Conventionally, when performing molecular-level analysis of biological samples, microscopic analysis of tissue morphology using an optical microscope or an electron microscope, and classical biochemical separation and fractionation methods have been mainstream approaches. However, in microscopic analysis, there has been a problem that the comprehensiveness and quantitativeness of observed molecules are inferior. In addition, in biochemical separation fractionation methods, freshness of the biological sample is important, and there has been a problem that the separation resolution is not sufficient since all separation operations need to be performed within a limited time in which the structure and activity of biological constitutional components can be maintained during the separation step of the sample.

For example, in fractionation of a biological sample, a so-called stepwise centrifugation method has been performed for a long time, in which the operation of separating the biological sample into a supernatant component and a precipitated component by centrifugation, and subjecting the obtained supernatant component to centrifugation by changing the centrifugal force stepwise is repeated in three stages, thereby obtaining fractions of cell nuclear components, organelle components, vesicle/macromolecule components, and cytoplasmic components. Furthermore, there has been an attempt to increase the number of steps in the stepwise centrifugation method in order to further enhance the fineness of separation. However, increasing the number of steps in the stepwise centrifugation method has limitations in the separation resolution of structures that can be separated. Moreover, density gradient centrifugation methods have been used for purification of viruses using cesium chloride density gradients and for purification of extracellular vesicles, mitochondria, and the like using sucrose density gradients.

However, when the purpose is to trace and capture all particles having various sizes and densities contained in a biological sample, it has been difficult to accurately recover all particles distributed along a continuous density gradient by existing density gradient centrifugation methods. Furthermore, when liquid chromatography, which is used for separation and fractionation of molecular groups of soluble components that do not precipitate even by ultracentrifugation methods, is employed for component analysis of biological samples, it is necessary to achieve sufficiently high separation resolution to separate different components into distinct peak fractions. On the other hand, as described above, since freshness of biological samples is important, there exists a time limitation. Accordingly, in the case of size-exclusion chromatography used for measuring and verifying particle sizes, even when using a highly precise long-size column employed for particle size analysis, it is not possible to secure sufficient separation resolution for adequately separating and evaluating, for example, monomers, dimers, and tetramers of albumin, which is a protein abundant in the living body. When an attempt is made to improve the separation resolution to adapt to biological samples containing substance components having a wide range of sizes, it is necessary to increase the column length. However, increasing the column length elevates the column pressure, thereby requiring a reduction in flow rate, which in turn increases the time required for separation analysis. Therefore, such methods are not suitable for rapid separation of biological samples.

Accordingly, an object of the present invention is to solve the above-described problems existing in the prior art and to provide a method for separating and fractionating, with high resolution, all components of a biological sample containing substance components of a wide range of sizes, while suppressing the time required for separation and fractionation.

### Solution to Problems

In view of the above object, the present invention provides a biological sample separation and fractionation method for separating and fractionating a biological sample, which is collected from a living body, into a plurality of fractions, the method including: a homogenization step of preparing a cell homogenate by adding a solvent to the biological sample and performing cell disruption; a first centrifugation step of subjecting the prepared cell homogenate to a centrifugation process at a first acceleration G1 to separate the prepared cell homogenate into a first supernatant component and a first pellet component; a stepwise centrifugation step of performing centrifugation (n-1) times by subjecting a centrifuge tube containing a separated (m-1)-th supernatant component to an m-th centrifugation process at an m-th acceleration Gₘ greater than an (m-1)-th acceleration Gₘ₋₁ to separate the (m-1)-th supernatant component into an m-th supernatant component and an m-th pellet component, where m is an integer of 2 to n, and repeating (n-1) times of separation of the (m-1)-th supernatant component; a density gradient centrifugal separation step of subjecting, to a density gradient centrifugation process, a predetermined amount of a density gradient sample prepared by adding the m-th pellet component to a density gradient reagent in a density gradient centrifuge tube, the density gradient reagent having a density gradient formed such that a density at a bottom portion of the density gradient centrifuge tube is higher than a density at an upper portion thereof; a density gradient centrifugal recovery step of recovering, in predetermined quantities according to a predetermined rule, the density gradient sample corresponding to the m-th pellet component in the density gradient centrifuge tube subjected to the density gradient centrifugal separation step, sequentially from an upper side or a bottom side, and fractionating the recovered density gradient sample into aₘ fractions, where aₘ is a predetermined integer corresponding to the m-th pellet component; and a chromatography step of adding the n-th supernatant component separated by the stepwise centrifugation step to a column of size-exclusion chromatography, subsequently supplying a buffer to the column at a predetermined flow rate to discharge, from the column, a chromatography sample in which the n-th supernatant component and the buffer are mixed, and recovering and fractionating the chromatography sample discharged from the column in predetermined quantities according to a predetermined rule into b fractions, where b is a predetermined integer, in which the entire biological sample is fractionated. It should be noted that, although the "acceleration Gₘ" means G1, G2, ..., Gn, that is, the acceleration G in the n-th centrifugation process, "m" is conveniently represented as a subscript because it is not possible to distinguish whether Gm-1 means [Gm] - 1 (subtracting 1 from Gm) or means G[m-1] (the (m-1)-th acceleration G).

In the above-described biological sample separation and fractionation method, the first centrifugation step is performed on the homogenate prepared from the biological sample, and subsequently, the stepwise centrifugation step is performed sequentially by performing centrifugation at incrementally increasing acceleration on the (m-1)-th supernatant component separated in the preceding centrifugation step. The density gradient sample prepared by adding the m-th pellet component separated in the stepwise centrifugation step to the density gradient reagent is subjected to the density gradient centrifugal separation step, and, in the subsequent density gradient centrifugal recovery step, the density gradient sample is recovered, in predetermined quantities according to a predetermined rule, sequentially from the upper side or the bottom side to separate the recovered density gradient sample into aₘ fractions. In the chromatography step, the n-th supernatant component finally obtained is subjected to size-exclusion chromatography to obtain the chromatography sample, which is recovered in predetermined quantities according to a predetermined rule and separated into b fractions. Each separation and fractionation method used in the above-described biological sample separation and fractionation method has the following disadvantages when the entire biological sample is to be analyzed. Simple centrifugation is intended to perform separation into two components, that is, a supernatant component and a pellet component, while stepwise centrifugation is suitable for rough fractionation such as separating nuclei having a large size, organelles having a medium size, and cytoplasm having a small size. However, the stepwise centrifugation has a limit on the number of separated fractions, and due to the principle of separation by centrifugal force, under the same solvent conditions, that is, under the same density of the solvent, each fraction inevitably contains a plurality of types of substances having little difference in densities or sizes. Therefore, a physiologically meaningful separation cannot be achieved. Furthermore, attempting to increase the number of fractions to be obtained to improve separation resolution solely by stepwise centrifugation requires increasing the number of times of repetitions of the centrifugation process, which takes a long time and is not suitable for a method of fractionating a biological sample that must be processed within a limited time. In addition, in the density gradient centrifugation, a centrifuge is used, and therefore, the sedimentation path length is limited by factors such as the size of the centrifuge device. Also, there exists an optimum range depending on the size and density of the substance to be separated, due to the characteristics of using density gradient media with varying densities. Therefore, when applying the above method to a sample containing a wide range of sizes, the separation resolution of the separated fractions decreases. In addition, chromatography, by the principle thereof, can be used only for fractionating substances of relatively small size, and is not suitable for fractionating an entire biological sample.

However, in the above-described biological sample separation and fractionation method according to the present invention, the m-th pellet components roughly divided based on size, particle diameter, and density are obtained in the stepwise centrifugation step, and them are subjected to the density gradient centrifugal separation step and the density gradient centrifugal recovery step, thereby making it possible to achieve fractionation with high separation resolution within a short time. Further, for the substances contained in the n-th supernatant component, which cannot be separated even by the stepwise centrifugation step, fractionation with high separation resolution can be achieved in a short time by subjecting the n-th supernatant component finally obtained through the stepwise centrifugation step to the size-exclusion chromatography process, recovering and fractionating the resulting chromatography sample in predetermined quantities according to a predetermined rule into b fractions.

In this way, by combining the stepwise centrifugation step, the density gradient centrifugal separation step and the density gradient centrifugal recovery step using density gradient centrifugal separation, and the chromatography step using size-exclusion chromatography, it becomes possible to subject the biological sample containing substances having a wide range of sizes, particle diameters, and densities to fractionation with high separation resolution, without omission and in a short time. Furthermore, since fractionation is achieved by preparing a density gradient sample from the m-th pellet component obtained in each stage of the stepwise centrifugation step, subjecting the prepared density gradient sample in the density gradient centrifuge tube to the density gradient centrifugal separation step, and then recovering the density gradient sample sequentially from the upper side or the bottom side of the density gradient centrifuge tube in the density gradient centrifugal recovery step, information on the sizes of the substances contained in each fraction is reflected in the recovery order of each fraction obtained in the density gradient centrifugal recovery step. Similarly, since fractionation is achieved by preparing the chromatography sample from the n-th supernatant component finally obtained through the stepwise centrifugation step, subjecting the prepared chromatography sample to the chromatography process, and sequentially recovering the prepared chromatography sample, information on the sizes of the substances contained in each fraction is also reflected in the recovery order of each fraction obtained in the chromatography step. Therefore, by analyzing the substances contained in each fraction, it becomes possible to obtain, in addition to information on the types of substances contained in each fraction, information on the sizes of the substances based on the recovery order of the separated fractions. Furthermore, since each fraction is recovered in predetermined quantities according to a predetermined rule, under the same conditions, each substance in the biological sample will be contained in the corresponding fraction with high reproducibility, and information on the size will also have high reproducibility.

In the above-described biological sample separation and fractionation method, it is preferable that, in the density gradient centrifugal recovery step corresponding to the m-th pellet component, the density gradient recovery reagent is evenly fractionated into aₘ fractions in amounts of xₘ ml each, and, in the chromatography step, the chromatography sample discharged from the column is evenly fractionated into b fractions in amounts of y ml each, where xₘ and y are predetermined integers, and it is more preferable to satisfy xₘ = y. By making the amounts of the respective fractions equal, it becomes possible to perform quantitative comparison of the amount of substances contained in the respective fractions among the plurality of fractions. For example, it becomes possible to compare which of a plurality of fractions obtained in the density gradient centrifugal recovery step contains a larger amount of a specific substance and which of a plurality of fractions obtained in the chromatography step contains a larger amount of a specific substance.

In the above case, when a density gradient centrifuge tube having a capacity of 14 ml is used, it is preferable that xₘ = y = 1 ml, aₘ = 14, and b = 30.

In one embodiment, n may be 3, and the three centrifugation processes may be performed.

For example, the density gradient reagent may be an iodixanol solution. In this case, it is preferable that a maximum value of a concentration of the density gradient reagent used when performing the density gradient centrifugal separation step on an (m+1)-th pellet component to be determined to be greater than a minimum value of a concentration of the density gradient reagent used when performing the density gradient centrifugal separation step on the m-th pellet component. For example, when n is 2, it is preferable that the density gradient reagent be formed to be layered by changing a concentration of the iodixanol solution in six stages, and it is more preferable that, in the density gradient reagent used when performing the density gradient centrifugal separation step on the second pellet component, the iodixanol solution be layered such that the concentrations thereof is 35%, 30%, 25%, 22.5%, 20%, and 15%, in order from a lower layer, and, in the density gradient reagent used when performing the density gradient centrifugal separation step on the third pellet component, the iodixanol solution be layered such that the concentrations thereof are 25%, 20%, 15%, 10%, 5%, and 0%, in order from a lower layer.

In addition, it is preferable that a plurality of columns connected in series be used in the chromatography step. In this case, for example, two columns connected in series may be used in the chromatography step.

In one embodiment, the fraction obtained in each step can be automatically separated and recovered by a fraction collector.

### Advantageous Effect of the Invention

According to the biological sample separation and fractionation method of the present invention, by combining the stepwise centrifugation step, the density gradient centrifugal separation step and the density gradient centrifugal recovery step using density gradient centrifugation, and the chromatography step using size-exclusion chromatography, it becomes possible to subject the biological sample containing substances having a wide range of sizes, particle diameters, and densities to fractionation with high separation resolution, without omission and in a short time. Moreover, information on the sizes of the substances contained in each fraction is reflected in each recovery order of the plurality of fractions obtained in each of the density gradient centrifugal recovery step and the chromatography step. Therefore, by analyzing the substances contained in each fraction, it becomes possible to obtain, in addition to the types of substances contained in each fraction, information on the sizes of the substances based on the recovery order of the separated fractions. Furthermore, since each fraction is recovered in predetermined quantities according to a predetermined rule, under the same conditions, each substance in the biological sample will be contained in the corresponding fraction with high reproducibility. Accordingly, it becomes possible to perform analysis such as size distribution and quantitative comparison of the substances contained in the biological sample, including which sizes are predominant.

### Brief Description of the Drawings

[FIG. 1] FIG. 1 is a flowchart showing a procedure of a biological sample separation and fractionation method for separating and fractionating a biological sample according to the present invention.
[FIG. 2] FIG. 2 is an explanatory diagram schematically illustrating an example of the procedure shown in FIG. 1.
[FIG. 3] FIG. 3 is a graph showing a distribution of the abundance of mitochondrial constituent proteins contained in each fraction obtained by the density gradient centrifugation and recovery step of the biological sample separation and fractionation method shown in FIG. 1.
[FIG. 4] FIG. 4 is a graph showing a distribution of the abundance of various proteins contained in each fraction separated by the chromatography step of the biological sample separation and fractionation method shown in FIG. 1.
[FIG. 5] FIG. 5 is a graph showing a size calibration curve representing a correlation between each fraction separated by the chromatography step of biological sample separation and fractionation method shown in FIG. 1 and the size of particles contained therein.
[FIG. 6] FIG. 6 is a graph showing a distribution of prion proteins of all sizes contained in each fraction, based on all fractions separated by the biological sample separation and fractionation method shown in FIG. 1.

### Description of Embodiments

With reference to the drawings, a biological sample separation and fractionation method for separating and fractionating a biological sample according to the present invention will be described below.

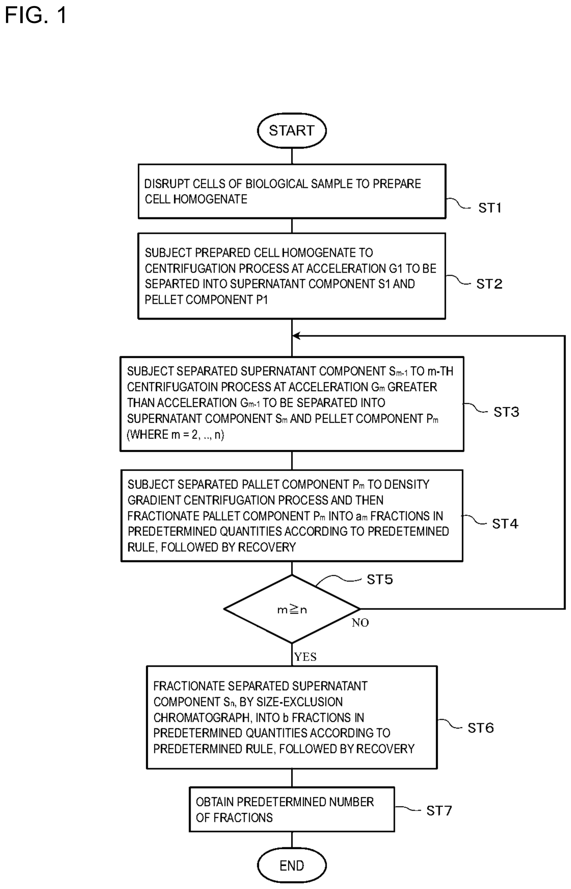

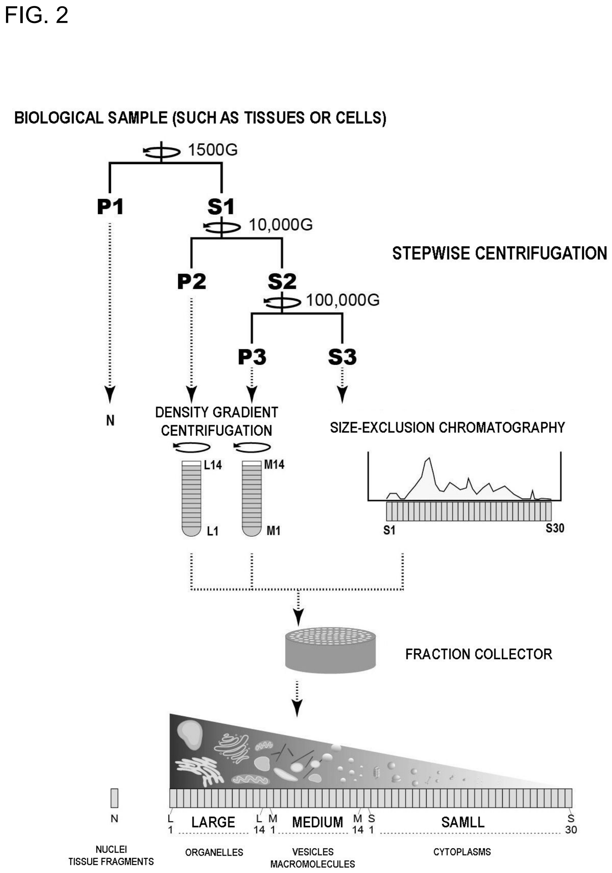

First, with reference to FIG. 1 and FIG. 2, the overall flow of the biological sample separation and fractionation method according to the present invention will be described.

First, a homogenization step is performed in which a biological sample, such as tissues or cells collected from a living body, of about 100 mg to 1 g is subjected to a homogenization operation (cell disruption operation) for about 5 minutes to prepare a cell homogenate (step ST1). This step releases intracellular organelles, macromolecules, and the like inside the tissues and cells of the biological sample. Next, a centrifuge tube containing the cell homogenate prepared in the step ST1 is subjected to a stepwise centrifugation step. Specifically, under an atmospheric pressure in a low temperature environment of about 2°C to 4°C, the cell homogenate prepared in the step ST1 is subjected to a first centrifugation process with an acceleration G1, thereby separating the cell homogenate into a first pellet component P1 and a first supernatant component S1 (step ST2). In the centrifugation process, coarse separation into the first pellet component P1 and the first supernatant component S1 is carried out by utilizing the principle that larger and heavier particles in the centrifuge tube sediment faster than smaller and lighter particles by applying a centrifugal force due to an acceleration to the centrifuge tube. For example, by performing the centrifugation process for about 5 minutes under a condition where the acceleration G1 is set to about 1,500 G, the cell homogenate can be separated into the first pellet component P1 and the first supernatant component S1. The first pellet component P1 obtained in the step ST2 is separated and then recovered as a nuclear fraction N. On the other hand, with respect to the first supernatant component S1 separated in the step ST2, in the step ST3, the centrifuge tube containing the first supernatant component S1 is subjected to a second centrifugation process at a centrifugal acceleration G2 greater than the centrifugal acceleration G1, thereby being further separated into a second pellet component P2 and a second supernatant component S2. Subsequently, the operation of subjecting the supernatant component obtained in this way in the preceding stage to a centrifugation process at an acceleration greater than that in the preceding centrifugation process, and further separating the supernatant component into a pellet component and a supernatant component is repeated a predetermined number of times. That is, in the step ST3, when m is an integer from 2 to n (m = 2, ..., n), the operation of subjecting the (m-1)-th supernatant component Sₘ₋₁ obtained by the (m-1)-th centrifugation process in the preceding stage to an m-th centrifugation process at an acceleration Gₘ greater than the acceleration Gₘ₋₁ in the (m-1)-th centrifugation process, and further separating the (m-1)-th supernatant component Sₘ₋₁ into an m-th pellet component Pₘ and an m-th supernatant component Sₘ is repeated until m = n, that is, until the n-th centrifugation process is performed. Therefore, in the step ST3, (n-1) pellet components are obtained.

In the stepwise centrifugation step, only rough separation into the pellet component and the supernatant component can be achieved. In the stepwise centrifugation step, since the centrifugal acceleration is increased stepwise, the pellet components obtained earlier contain relatively large and heavy substances that are likely to sediment, while the pellet components obtained later contain relatively small and light substances that are less likely to sediment. Therefore, in order to perform fractionation with sufficient separation resolution by the centrifugation process alone, it would be necessary to perform a large number of centrifugation processes in the stepwise centrifugation step. However, the freshness of the biological sample is important, and in order to maintain the structure and activity of the biological constitutional components and to suppress oxidation and degradation reactions of the molecular groups during the separation and fractionation process, it is desirable to complete the separation and fractionation process of the biological sample (sample such as tissues or cells), that is, the operations from disruption of the biological sample to all separation and recovery steps, within 10 hours in a biological solution and under a low temperature condition of 2°C to 4°C. Therefore, it is not practical to perform a large number of centrifugation processes. Furthermore, centrifugation process is, in terms of the principle thereof, effective for separation when there is a large difference in sedimentation coefficients, which are indicators of the ease of sedimentation due to gravity. A stepwise centrifugation process is also suitable for separation into approximately three groups, such as nuclei having large particle sizes, organelles having medium particle sizes, and cytoplasm of fine particles. However, it is not suitable for separation when the difference in sedimentation coefficients is small. Consequently, a large number of substances are mixed in the pellet component, which do not result in physiologically meaningful separations. Accordingly, in the biological sample separation and fractionation method according to the present invention, the m-th pellet component Pₘ (excluding the first pellet component P1) obtained at each stage of the stepwise centrifugation step is subjected to density gradient centrifugation, and is further separated finely according to density differences of the substances contained in the m-th pellet component Pₘ.

It is known that, in the stepwise centrifugation method, performing stepwise centrifugation three times can achieve separation into three rough groups: nuclear components such as nuclei and tissue fragments, large organelles and the like, and small cytoplasms such as vesicles and the like. Considering that further separation and fractionation are performed by the density gradient centrifugal separation step in the latter stage of the biological sample separation and fractionation method according to the present invention, and that, as described above, it is desirable to complete the entire separation and fractionation process in a short time, it can be said that separation into such three groups is sufficient in the stepwise centrifugation step. Therefore, in the biological sample separation and fractionation method according to the present invention, n is preferably three or more, and more preferably, n = 3. In the biological sample separation and fractionation method according to the present invention, when three stages of centrifugation processes, including the step ST2, are performed with n = 3, it is general that the first pellet component P1 contains nuclear components such as nuclei and tissue fragments, the second pellet component P2 contains large organelles (cellular organelles), and the third pellet component contains small organelles, vesicles, macromolecules, and the like.

Among the pellet components obtained at each stage of the stepwise centrifugation step of the step ST3, the m-th pellet component Pₘ, excluding the first pellet component P1, is subjected to a density gradient centrifugal separation step in the step ST4, and thereafter, in the density gradient centrifugal recovery step, is separated and recovered into aₘ fractions from either the upper side or the bottom side of the tube, in predetermined quantities according to a predetermined rule. Here, aₘ is a predetermined integer corresponding to the m-th pellet component. Specifically, in a density gradient centrifuge tube, a density gradient reagent is formed so that the density at the bottom portion is higher than that at the top portion, and each pellet component Pₘ obtained in the stepwise centrifugation step (excluding the first centrifugation step) is added to the density gradient reagent to prepare a density gradient sample in a predetermined amount. The maximum value of the concentration range of the density gradient reagent used when performing the density gradient centrifugal separation step on the (m+1)-th pellet component is set to be greater than the minimum value of the concentration range of the density gradient reagent used when performing the density gradient centrifugal separation step on the m-th pellet component. The amount of the prepared density gradient sample may be appropriately determined in consideration of the capacity of the density gradient centrifuge tube used, such that the total amount of the desired fractions can be recovered. The density gradient centrifuge tube containing the density gradient sample is subjected to a centrifugation process at a predetermined acceleration using a centrifugation device. As a result, a plurality of substances contained in the m-th pellet component Pₘ sediment to predetermined positions in the density gradient reagent according to the densities of the substances, thereby being separated. In this case, the substances having greater densities tend to be positioned closer to the bottom side. After completion of the density gradient centrifugal separation step for the m-th pellet component Pₘ, in the density gradient centrifugal recovery step, for example, a hole is made at the bottom portion of the density gradient centrifuge tube, and while a buffer (that is, 0% density gradient reagent) is added to the uppermost surface of the density gradient sample in an amount equal to the recovery flow rate, the density gradient sample in the density gradient centrifuge tube is discharged sequentially from the bottom side at the predetermined recovery flow rate, and separated and recovered into aₘ fractions in predetermined quantities according to a predetermined rule. Alternatively, the density gradient centrifugal recovery step may be performed, for example, by inserting a suction device into the density gradient sample from the upper side of the density gradient centrifuge tube and aspirating the density gradient sample sequentially from the bottom side, thereby separating and recovering the density gradient sample sequentially from the bottom side in predetermined quantities according to a predetermined rule. Here, the amount of the density gradient sample recovered for each fraction includes both an amount of buffer having a volume equivalent to a flow passage exclusion volume of a flow passage required for discharge from the density gradient centrifuge tube and an amount of buffer added during recovery. The amount of each fraction may be appropriately determined so that physiologically meaningful fractionation can be achieved, taking into account the diameter and the sedimentation path length of the density gradient centrifuge tube to be used. For example, when a centrifuge tube having a sedimentation path length of 9 to 10 cm and a capacity of 14 ml is used as the density gradient centrifuge tube, it is preferable that the amount of each fraction is about 1 ml.

When performing the density gradient centrifugal recovery step according to the procedure as described above, the density gradient sample is discharged sequentially from the bottom side of the density gradient centrifuge tube that has been subjected to the centrifugation process. Therefore, the densities, that is, the sizes, of the substances contained in each fraction decrease according to the order in which the fractions are taken out. Accordingly, it is possible to obtain information concerning the size of a target substance contained in each fraction from the order in which the fractions are taken out. Furthermore, since each density gradient sample is separated into the respective fractions in predetermined quantities according to a predetermined rule, each substance contained in each density gradient sample is separated into a corresponding fraction with high reproducibility under the same conditions. This makes it possible to compare the sizes of the substances contained in each fraction based on the order in which the fractions are recovered.

When the density gradient sample is discharged from the bottom portion of the density gradient centrifuge tube by forming a hole in the bottom portion of the density gradient centrifuge tube, it is preferable to connect the hole at the bottom portion of the density gradient centrifuge tube to a fraction collector through an intermediate tube, and to automatically separate and recover the density gradient sample in predetermined quantities using the fraction collector. This allows the density gradient sample subjected to the density gradient centrifugal separation step to be efficiently separated and recovered in accurate quantities and in a short time. In particular, in order to obtain information on the size of the substance contained in each fraction based on the order in which the fractions are recovered, it is necessary to reproducibly recover each substance contained in the density gradient sample into the corresponding fraction, and accurate fractionation by quantity is important. From this viewpoint, it is preferable to separate and recover the density gradient sample subjected to the density gradient centrifugal separation step using a fraction collector.

However, the density gradient centrifugal recovery step is not limited to the above-described procedure, provided that the order in which each fraction is recovered is associated with the density, that is, the size, of the substances contained in each fraction. For example, the density gradient centrifugal recovery step may be performed by inserting a suction device from the upper side of the density gradient centrifuge tube and aspirating the density gradient sample sequentially from the upper side thereof, thereby separating and recovering the density gradient sample from the upper side in predetermined quantities according to a predetermined rule. In this case, since the density gradient sample is recovered sequentially from the upper side of the density gradient centrifuge tube that has been subjected to the centrifugation process, the densities, that is, the sizes, of the substances contained in each fraction increase in accordance with the order in which the fractions are taken out, in contrast to recovery from the bottom side. Accordingly, in order that the sizes of the substances contained in the respective fractions decrease in accordance with the order of arrangement of the fractions, the fractions may be arranged in the reverse order of recovery.

It is noted that known density gradient reagents such as cesium chloride or sucrose can be used as the density gradient reagent; however, it is preferable to use iodixanol or the like, which does not affect the osmotic pressure of the biological sample. In the initially formed density gradient, the density change may be either continuous or stepwise. In the case of stepwise density changes, the density gradient may be formed by layering density gradient reagents such that the density gradient reagent with the highest density is arranged at the bottom of the density gradient centrifuge tube and density gradient reagents of decreasing densities are sequentially layered toward the upper portion. When Iodixanol is used as the density gradient medium, even if the density gradient reagents are layered in a layered manner, a continuous density gradient in which the density continuously changes is formed during the centrifugation process. For the density gradient centrifuge tube, a density gradient centrifuge tube having a sedimentation path length of about 5 cm and a capacity of about 5 ml, a density gradient centrifuge tube having a sedimentation path length of about 9 to 10 cm and a capacity of about 14 ml, and a density gradient centrifuge tube having a sedimentation path length of about 9 to 10 cm and a capacity of about 17 to 40 ml are generally used. From the principle of centrifugation, the longer the sedimentation path length, the greater the difference in sedimentation speed, resulting in higher separation resolution, and the narrower the tube diameter, the smaller the processing volume and the shorter the time required for separation and recovery, that is, the higher the recovery efficiency. Therefore, from the standpoint of balancing separation resolution and recovery efficiency, a thin and long density gradient tube is desirable, and it is preferable to use a density gradient centrifuge tube having a sedimentation path length of about 9 to 10 cm and a capacity of about 14 ml.

It is preferable that the amount of all fractions obtained in the density gradient centrifugal recovery step corresponding to the m-th pellet component Pₘ be equal to the same, xₘ ml. This is because analytical results among fractions, for example, quantitative comparisons of the substances contained in the respective fractions, can be performed between the respective fractions. For example, it becomes possible to compare which fraction contains a greater amount of a specific substance among a plurality of fractions obtained in the density gradient centrifugal recovery step. For the same reason, it is more preferable that the amount of all fractions obtained in the density gradient centrifugal recovery step performed for all m-th pellet components Pₘ (m = 2, ..., n) be the same, xₘ = x ml. When a density gradient centrifuge tube having a sedimentation path length of about 9 to 10 cm and a capacity of about 14 ml is used and the amounts of all fractions obtained in the density gradient centrifugal recovery step are made equal, the amount of each fraction is preferably 1 ml. By determining the amount of each fraction in this way, sufficient separation resolution can be secured for identifying the types of substances to be analyzed.

When the n-th centrifugation process is completed in the step ST3 (step ST5), the size-exclusion chromatography process is performed on the n-th supernatant component Sn, and a chromatography sample obtained by the size-exclusion chromatography process is separated and recovered into b pieces of fractions, in predetermined quantities according to a predetermined rule (step ST6). Here, b is a predetermined integer. Specifically, the n-th supernatant component Sn separated by the n-th centrifugation process is added to a size-exclusion separation column using an autosampler or the like, and thereafter a buffer is supplied to the size-exclusion separation column at a predetermined flow rate, whereby the n-th supernatant component Sn flows through the interstices of the packing material in the size-exclusion separation column together with the buffer, and the substances contained in the n-th supernatant component Sn are discharged from the size-exclusion separation column as the chromatography sample together with the buffer over times corresponding to their sizes. The chromatography sample containing the n-th supernatant component Sn is a chromatography sample obtained after a buffer having a volume equivalent to the exclusion volume of the size-exclusion separation column is discharged from the size-exclusion separation column and until a buffer having a volume equivalent to the column volume is discharged. The chromatography sample discharged from the size-exclusion separation column is recovered while being separated, in the order of discharge, into each fraction in predetermined quantities according to a predetermined rule.

The size-exclusion separation column used in size-exclusion chromatography is packed with a packing material having numerous pores. Smaller molecules flow while permeating deeper into the pores of the packing material, thereby increasing the time required before the molecules are discharged from the size-exclusion separation column. By virtue of this characteristic, separation of substances is achieved by utilizing the fact that the time required for discharge differs according to the size of the molecule. When the chromatography sample is separated and recovered into each fraction in predetermined quantities according to a predetermined rule, in the order of discharge from the size-exclusion separation column, those substances in the chromatography sample that have larger sizes are contained in fractions recovered earlier, whereas those substances in the chromatography sample that have smaller sizes are contained in fractions recovered later. Accordingly, information on the sizes of the substances contained in each fraction can be obtained from the order in which the fractions are recovered. Furthermore, since the chromatography sample is separated into each fraction in predetermined quantities according to a predetermined rule, respective substances contained in the chromatography sample are reproducibly separated into corresponding fractions under the same conditions, and the sizes of the substances contained in respective fractions can be compared based on the order in which the fractions are recovered.

It is preferable that the separation and recovery of the chromatography sample discharged from the size-exclusion separation column be performed by connecting the size-exclusion separation column to a fraction collector through an intermediate tube and using the fraction collector. This makes it possible to efficiently separate and recover, in accurate quantities and in a short time, the chromatography sample discharged from the size-exclusion separation column. In particular, in order to obtain information on the sizes of the substances contained in each fraction based on the order in which each fraction is recovered, it is necessary that each substance contained in the chromatography sample be reproducibly recovered into a corresponding fraction, and accurate fractionation by quantity is important. From this viewpoint, it is preferable that the separation and recovery of the chromatography sample discharged from the size-exclusion separation column be performed using a fraction collector.

Moreover, since, according to the above-described principle, the time taken until the substances contained in the n-th supernatant component are discharged from the size-exclusion column varies, it is preferable to increase the travel distance of the sample, that is, to lengthen the size-exclusion separation column in order to enhance separation resolution. However, in size exclusion chromatography, it is required to forcibly feed a buffer into a size-exclusion separation column by the principle of size-exclusion chromatography, whereas there is an upper limit to the pressure that can be applied to the size-exclusion separation column due to the strength of the size-exclusion separation column. Accordingly, there is also an upper limit to the flow rate of the buffer that flows through the size-exclusion separation column. When the length of the size-exclusion separation column is increased in order to improve separation resolution, the pressure required to feed the buffer into the size-exclusion separation column increases, and the pressure acting on the size-exclusion separation column also increases. Therefore, it is necessary to reduce the flow rate at which the buffer is fed into the size-exclusion separation column. As a result, the time required for the chromatography step increases, which hinders rapid separation of the biological samples. Therefore, in the chromatography step of the biological separation and fractionation method according to the present invention, it is preferable that a plurality of size-exclusion separation columns be connected in series. This makes it possible to suppress an increase in pressure acting on any one size-exclusion separation column and to improve separation resolution while maintaining a flow rate to suppress an increase in processing time. Furthermore, it is preferable to employ a size-exclusion separation column having high pressure resistance so that the flow rate of the buffer can be made as high as possible.

It should be noted that, when the size-exclusion separation columns are connected in series, the path through which the chromatography sample flows becomes longer, so that the time required until all substances contained in the n-th supernatant component added to the first size-exclusion separation column are discharged from the last size-exclusion separation column becomes longer. Considering the balance between the desirability that biological samples, for which freshness is important, be processed in as short a time as possible and the desirability of securing high separation resolution, it is preferable to perform the chromatography step by connecting, for example, two size-exclusion separation columns each having a length of 30 cm in series.

It is preferable that the quantity, predetermined according to a predetermined rule, for each fraction in the chromatography step be the same, y ml, for all fractions. This is because, similarly to the case of the density gradient centrifugal recovery step, analytical results among fractions, for example, the quantitative comparisons of the substances contained in the respective fractions, can be performed between the respective fractions. For example, it becomes possible to compare which of a plurality of fractions obtained in the chromatography step contains a greater amount of a specific substance.

When the quantities of the fractions recovered from all the pellet components excluding the first pellet component in the density gradient centrifugal recovery step are all made the same x ml, and the quantities of the fractions recovered from the supernatant components in the chromatography step are all made the same y ml, it is preferable to satisfy x = y. This significantly simplifies the analytical process and facilitates quantitative comparison of the substances contained in all the fractions obtained by the biological sample separation and fractionation method according to the present invention.

As described above, in the biological sample separation and fractionation method according to the present invention, first, the biological sample is roughly divided into a plurality of pellet components and a supernatant component according to the sizes of the substances contained in the biological sample by the stepwise centrifugation process, each pellet component, in which the range of the sizes of the substances contained is limited, is, except for the pellet component obtained by the first centrifugation process, separated and recovered into a plurality of fractions according to density by density gradient centrifugation process, and the supernatant component remaining at the end is separated and recovered into a plurality of fractions according to size by size-exclusion chromatography process. As a result, all the substances contained in the biological sample are separated and fractionated into [1 + (n-1)·a + b] fractions (step ST7). Further, in the stepwise centrifugation step, since the acceleration during centrifugation is increased stepwise, the first pellet component P1 obtained by the first centrifugation process contains larger and heavier substances than the m-th pellet component Pₘ obtained by subsequent centrifugation processes. Accordingly, the range of sizes, weights, and the like of the substances contained in the m-th pellet component Pₘ is narrower than that of the initial biological sample. Therefore, when density gradient centrifugation process is performed for each pellet component, a high separation resolution can be secured even when a short density gradient centrifuge tube is used. Moreover, by using a short density gradient centrifuge tube, separation and fractionation process can be performed in a short time. As a result, high separation resolution can be achieved within a short time that can preserve the freshness of the biological sample. It should be noted that, since the size-exclusion chromatography process is performed on the n-th supernatant component Sn finally obtained by the stepwise centrifugation step, the n-th supernatant component Sn contains only sufficiently small substances, and thus there is no concern of clogging the size-exclusion separation column.

In addition, by using a fraction collector for separation and fractionation, not only can the time required for separation and fractionation be significantly shortened, but fractionation in accurate quantities becomes possible. When fractionation is thus performed in accurate quantities, the respective substances will be reproducibly contained in the corresponding fractions under the same conditions. In addition, when fractionation is performed with sufficient separation resolution, substances having different sizes are contained in different fractions. As a result, if the fractions differ, it can be understood that the sizes of the substances contained in the respective fractions also differ, and analysis becomes possible as to, even for the same molecule, whether monomers of the molecule are abundant or dimers of the molecule are abundant, and the like.

In the above description, the "acceleration Gₘ" means G1, G2, ..., Gn, that is, the acceleration G in the n-th centrifugation process. However, since it is not possible to distinguish whether Gm-1 means [Gm]-1 (subtracting 1 from Gm) or G[m-1] (the (m-1)-th acceleration G), the symbol "m" is conveniently expressed as a subscript. The same applies to the expressions "m-th pellet component Pₘ" and "m-th supernatant component Sₘ".

Next, one embodiment of the biological sample separation and fractionation method according to the present invention will be described below.

First, a biological sample of about 100 mg to 1 g, such as a tissue collected from a living body, is subjected to a disruption process for about 5 minutes together with a solvent using a homogenizer, thereby solubilizing the biological sample to prepare a cell homogenate. Next, a centrifuge tube containing the cell homogenate is subjected to a stepwise centrifugation step (centrifugation step) three times under atmospheric pressure in a low temperature environment of about 2°C to 4°C. Specifically, in the first centrifugation step, cooling centrifugation is performed twice at an acceleration of 1,500 G for 5 minutes; in the second centrifugation step, cooling centrifugation is performed at an acceleration of 10,000 G for 20 minutes; and in the third centrifugation step, cooling centrifugation is performed at an acceleration of 100,000 G for 60 minutes. By performing centrifugation according to such a procedure, the entire stepwise centrifugation step, including a preparation operation, a recovery operation, and the like, can be performed in about 2 hours. As the centrifuge tube, a centrifuge tube having a sedimentation path length of about 9 to 10 cm and a capacity of 14 ml is used.

In the first centrifugation step, the cell homogenate is separated into a first pellet component P1 and a first supernatant component S1. The first pellet component P1 obtained in the first centrifugation step mainly contains nuclei and cell fragments, and is recovered as a nuclear component N1. On the other hand, the first supernatant component S1 obtained in the first centrifugation step is placed in a centrifuge tube and then is further separated into a second pellet component P2 and a second supernatant component S2 by the second centrifugation step under the above-described conditions. Furthermore, the second supernatant component S2 obtained in the second centrifugation step is placed in a centrifuge tube and then is further separated into a third pellet component P3 and a third supernatant component S3 by the third centrifugation step under the above-described conditions. Thus, in the stepwise centrifugation step, the biological sample is separated into the first pellet component P1, the second pellet component P2, the third pellet component P3, and the third supernatant component S3.

Next, density gradient centrifugal separation step is performed for the second pellet component P2 and the third pellet component P3. First, with respect to the second pellet P2, using iodixanol as a density gradient reagent, a first density gradient sample of a predetermined amount is prepared in a first density gradient centrifuge tube having a sedimentation path length of about 9 to 10 ml and a capacity of 14 ml by layering, in the following order, from the bottom of the first density gradient centrifuge tube: 1 ml of 35% iodixanol; a mixture of 200 µl of 50% iodixanol and 100 µl of the second pellet component P2; 1 ml of 30% iodixanol; 2 ml of 25% iodixanol; 2 ml of 22.5% iodixanol; 2 ml of 20% iodixanol; and 2 ml of 15% iodixanol. The prepared first density gradient sample is subjected to centrifugation process at an acceleration of 100,000 G for 2 hours under atmospheric pressure in a low temperature environment of 2°C to 4°C. After completion of the centrifugation process for the first density gradient centrifuge tube containing the first density gradient sample subjected to the centrifugation process, a hole is made in the bottom portion of the first density gradient centrifuge tube, and a fraction recovery system is connected to the bottom portion of the first density gradient centrifuge tube, while a peristaltic pump is connected to a downstream side of the fraction recovery system. In order to prevent air from being mixed, a buffer (that is, 0% density gradient reagent) is added to the uppermost surface of the first density gradient sample at a rate of 1 ml/min, while the first density gradient sample in the first density gradient centrifuge tube is aspirated sequentially from the bottom portion of the first density gradient centrifuge tube at a flow rate of 1 ml/min. Alternatively, without making a hole in the bottom portion of the first density gradient centrifuge tube, the first density gradient sample may be aspirated sequentially from the bottom side by inserting a suction device from the upper portion of the first density gradient centrifuge tube. The aspirated first density gradient sample is separated by 1 ml increments with a fraction collector connected to the downstream side of the peristaltic pump, then fractionated into 14 fractions, through L1 to L14, and recovered. Here, the first density gradient sample recovered in the fractions L1 to L14 is considered to include an amount of buffer having a flow passage exclusion volume of a flow passage for discharge from the density gradient centrifuge tube and an amount of buffer added during recovery. The fractions L1 to L14 mainly contain particle groups of large size, such as organelles. The fractions L1 to L14 are designated in correspondence with the order in which the fractions are recovered from the bottom portion of the first density gradient centrifuge tube.

It should be noted that the recovery of the first density gradient sample from within the first density gradient centrifuge tube may alternatively be performed by inserting a suction device from the upper side of the first density gradient centrifuge tube and aspirating the first density gradient sample sequentially from the upper side thereof, thereby separating and recovering the first density gradient sample from the upper side in predetermined quantities according to a predetermined rule. In this case, since the first density gradient sample is recovered sequentially from the upper side of the density gradient centrifuge tube that has been subjected to the centrifugation process, the densities, that is, the sizes, of the substances contained in each fraction increase in accordance with the order in which the fractions are taken out, in contrast to recovery from the bottom side. Accordingly, in order that the sizes of the substances contained in the respective fractions decrease in accordance with the order of arrangement of the fractions, the fractions may be separated and recovered in reverse order from the fraction L14 to the fraction L1, opposite to the case where the first density gradient sample is recovered sequentially from the bottom side.

Similarly, with respect to the third pellet component P3, using iodixanol as the density gradient reagent, a second density gradient sample of a predetermined amount is prepared in a second density gradient centrifuge tube having a sedimentation path length of about 9 to 10 cm and a capacity of 14 ml by layering, in the following order, from the bottom portion (lower layer) of the second density gradient centrifuge tube: 1 ml of 25% iodixanol; 2 ml of 20% iodixanol; a mixture of 400 µl of 25% iodixanol and 100 µl of the third pellet component P3; 2 ml of 15% iodixanol; 2 ml of 10% iodixanol; 2 ml of 5% iodixanol; and 1.5 ml of 0% iodixanol. The prepared second density gradient sample is subjected to centrifugation process at an acceleration of 100,000 G for 2 hours under atmospheric pressure in a low temperature environment of 2°C to 4°C. After completion of the centrifugation process for the second density gradient centrifuge tube containing the second density gradient sample subjected to the centrifugation process, a hole is made in the bottom portion of the second density gradient centrifuge tube using a fraction recovery system, and the fraction recovery system is connected to the bottom portion of the second density gradient centrifuge tube, while a peristaltic pump is connected to a downstream side of the fraction recovery system. In order to prevent air from being mixed, a buffer (that is, 0% density gradient reagent) is added to the uppermost surface of the second density gradient sample at a rate of 1 ml/min, while the second density gradient sample in the second density gradient centrifuge tube is aspirated sequentially from the bottom portion of the second density gradient centrifuge tube at a flow rate of 1 ml/min . As in the case of the first density gradient centrifuge tube, the second density gradient sample may also be aspirated sequentially from the bottom side by inserting a suction device from the upper portion of the second density gradient centrifuge tube without making a hole in the bottom portion of the second density gradient centrifuge tube. The aspirated second density gradient sample is separated by 1 ml increments with a fraction collector connected to the downstream side of the peristaltic pump, then fractionated into 14 fractions, through M1 to M14, and recovered. Here, the second density gradient sample recovered in the fractions M1 to M14 is considered to include an amount of buffer having a flow passage exclusion volume of a flow passage for discharge from the density gradient centrifuge tube and an amount of buffer added during recovery. The fractions M1 to M14 mainly contain particle groups of medium size, such as small organelles, vesicles, and macromolecules. The fractions M1 to M14 are designated in accordance with the order in which the fractions are recovered from the bottom portion of the second density gradient centrifuge tube.

It should be noted that, as in the case of the first density gradient centrifuge tube, the recovery of the second density gradient sample from within the second density gradient centrifuge tube may alternatively be performed by inserting a suction device from the upper side of the second density gradient centrifuge tube and aspirating the second density gradient sample from the upper side thereof, thereby separating and recovering the second density gradient sample from the upper side in predetermined quantities according to a predetermined rule. In this case, since the second density gradient sample is recovered sequentially from the upper side of the second density gradient centrifuge tube that has been subjected to the centrifugation process, the densities, that is, the sizes of the substances contained in each fraction increase in accordance with the order in which the fractions are taken out, in contrast to recovery from the bottom side. Accordingly, in order that the sizes of the substances contained in the respective fractions decrease in accordance with the order of arrangement of the fractions, the fractions may be separated and recovered in reverse order from the fraction L14 to the fraction L1, opposite to the case where the second density gradient sample is recovered sequentially from the bottom side.

Since the process for the second density centrifuge tube and the third density centrifuge tube can be performed in parallel, the density gradient centrifugal separation step and the density gradient centrifugal recovery step, including a preparation operation, a recovery operation, and the like, can be performed in about 4 hours by performing the operations according to the above-described procedure.

With respect to the third supernatant component finally obtained by the third centrifugation step, the chromatography step is performed. First, about 500 µl of the third supernatant component S3 obtained by the third centrifugation step is added to a column which is constituted by connecting, in series, two size-exclusion separation columns (capable of withstanding a high pressure of 1.0 MPa or more) each having an inner diameter of 10 mm, a length of 300 mm, and a column volume of 24 ml (hereinafter, simply referred to as a series-connected size-exclusion separation column), and thereafter buffer is supplied to the series-connected size-exclusion separation column at a flow rate of 0.5 ml/min. Then, the portion from the time when the buffer having a volume equivalent to the exclusion volume of the series-connected size-exclusion separation column is discharged until the time when the buffer having a volume equivalent to the column volume of the series-connected size-exclusion separation column is discharged constitutes a chromatography sample in which the substances contained in the third supernatant component are included in the buffer discharged from the size-exclusion separation column. Here, the chromatography sample has a volume of 30 ml. This chromatography sample of the 30 ml is separated by 1 ml increments with a fraction collector, and are fractionated and recovered into 30 fractions, through S1 to S30. The fractions S1 to S30 contain small cytoplasm and the like. The fractions S1 to S30 are designated in correspondence with the order in which the fractions are discharged from the size-exclusion separation column.

In this way, a total of 59 fractions of N1, L1 to L14, M1 to M14, and S1 to S30 are obtained. These 59 fractions are sufficient and necessary for detailed evaluation of the localization distribution of various organelles having different sizes and types as well as changes in the sizes of particles and molecules. In addition, according to the above-described method, all separation and fractionation processes can be completed within 10 hours, which is sufficient to maintain the freshness of the biological sample. Furthermore, the fractions L1 to L14, M1 to M14, and S1 to S30 are arranged in this order such that the sizes of the substances contained in the respective fractions become progressively smaller.

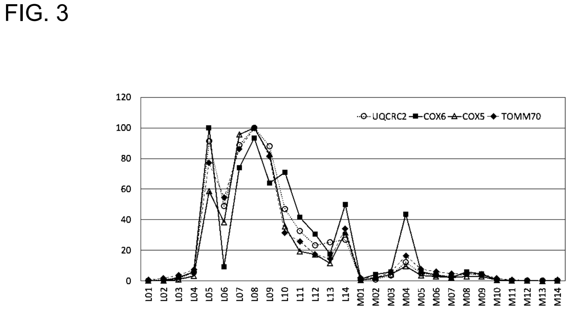

FIG. 3 shows the results of the analysis based on the fractions L1 to L14 and M1 to M14 obtained by the density gradient centrifugal separation step and the density gradient centrifugal recovery step, which are performed for the second pellet component P2 and the third pellet component P3 in the procedure as described above, and is a graph illustrating the distribution of the abundance of the mitochondrial constituent proteins. In the graph shown in FIG. 3, the horizontal axis represents the fractions L1 to L14 recovered from the first density gradient sample derived from the second pellet component P2 obtained by the second centrifugation step, and the fractions M1 to M14 recovered from the second density gradient sample derived from the third pellet component P3 obtained by the third centrifugation step, which are arranged in order of recovery. This horizontal axis corresponds to an index of the size of particles contained in each fraction and indicates that the size of the particles decreases from left to right in the figure. The vertical axis represents relative values of the signal intensities measured by a mass spectrometer for the respective fractions, when a maximum value of a signal intensity measured by the mass spectrometer for a target protein is defined as 100. In FIG. 3, the symbol "o (white circle)" represents the measurement results for the mitochondrial constituent protein UQCRC2; the symbol "▪ (black square)" represents the measurement results for the mitochondrial constituent protein COX6; the symbol "△ (white triangle)" represents the measurement results for the mitochondrial constituent protein COX5; and the symbol "blackened◇ (diamond)" represents the measurement results for the mitochondrial constituent protein TOMM70. For each mitochondrial constituent protein, peaks of signal intensity appear at different fractions, indicating that even the same protein exists in various molecular sizes. Furthermore, it can be understood that, if it is assumed that the number of fractions in the density gradient centrifugal recovery steps is halved to 7, for example, with respect to various mitochondrial constituent proteins, the fractions L5 and L6, and the fractions L7 and L8, respectively, would be merged into single fractions, and therefore, it might become impossible to exhibit two peaks as shown in FIG. 3. Therefore, it is preferable that the number of fractions aₘ in the density gradient centrifugal recovery step satisfy aₘ ≥ 7, and more preferably aₘ ≥ 14.

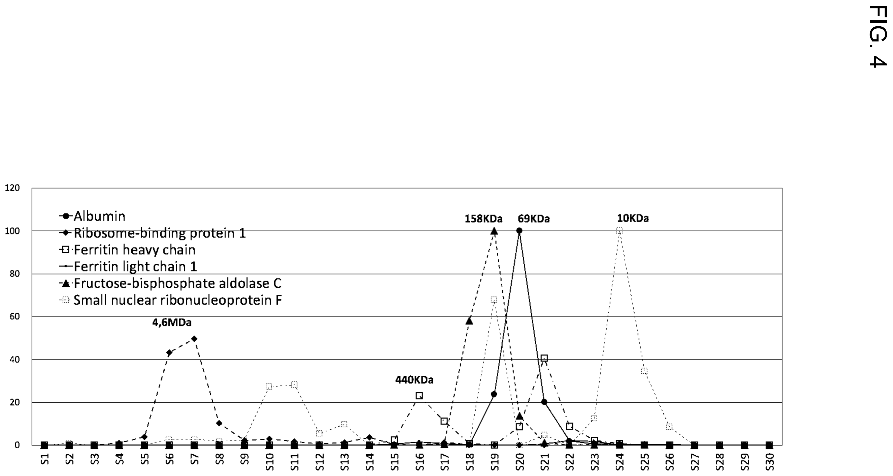

FIG. 4 shows the results of the analysis based on the fractions S1 to S30 obtained by the chromatography step performed for the third supernatant component S3 in the procedure as described above, and is a graph illustrating the distribution of the abundance of several substances contained in the supernatant component S3 derived from the biological sample. In the graph shown in FIG. 4, the horizontal axis represents the fractions S1 to S30 recovered from the chromatography sample derived from the third supernatant component S3 obtained by the third centrifugation process, which are arranged in order of recovery. This axis corresponds to an index of the size of the particles contained in each fraction and indicates that the size of the particles decreases from left to right in the figure. The vertical axis represents relative values of the signal intensities measured by a mass spectrometer for the respective fractions when a maximum value of a signal intensity measured by the mass spectrometer for a target protein is defined as 100. In FIG. 4, the symbol "▲ (black triangle)" represents the measurement results for Albumin; the symbol "blackened ◇(diamond)" represents the measurement results for Ribosome-binding protein; the symbol "o(white square)" represents the measurement results for Ferritin heavy chain; the symbol "• (black circle)" represents the measurement results for Ferritin light chain; the symbol "▲ (black triangle)" represents the measurement results for Fructose-bisphosphate aldolase C; and the symbol "dashed □ (square)" represents the measurement results for Small nuclear ribonucleoprotein F.

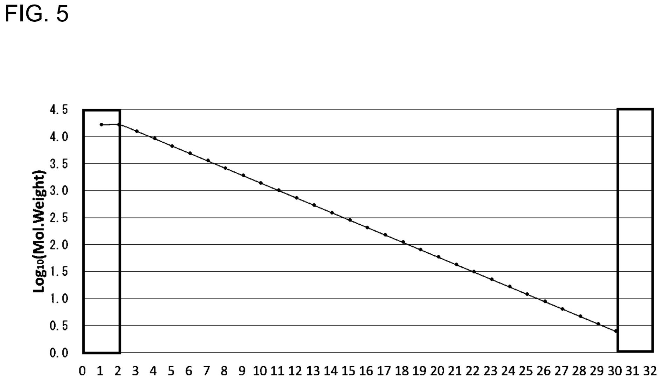

FIG. 5 is a graph showing a size calibration curve, indicating the correlation between each fraction S1 to S30 obtained in the chromatography step and the molecular weight of the substance contained therein, where the size calibration curve is obtained using standard substances of known sizes. The horizontal axis represents the fractions S1 to S30 obtained in the chromatography step, which are arranged in order of recovery, and the vertical axis represents the molecular weight on a common logarithmic scale. When such a size calibration curve as shown in FIG. 5 is prepared in advance, the size of the substances contained in each fraction S1 to S30 can be estimated from the results shown in FIG. 4. The numerical values described near the peaks in FIG. 4 represent the molecular weights of the corresponding fractions as determined from the size calibration curve.

From FIG. 4, it can be seen that, for each substance, peaks of signal intensity appear at different fractions, indicating that the substances were separated and recovered with high accuracy. Furthermore, in FIG. 4, when the number of fractions recovered in the chromatography step is 30, Fructose-bisphosphate aldolase C exhibits a peak in the fraction S19, Albumin exhibits a peak in the fraction S20, and from the size calibration curve shown in FIG. 5, their molecular weights are determined to be 158 kDa and 69 kDa, respectively. It can be understood that, if the number of fractions in the chromatography step is halved to 15, the substances that are separated into the fractions S19 and S20 in the case shown in FIG. 4 would be separated into a single fraction, making it impossible to detect a twofold change in size for a protein of about 70 kDa. Furthermore, it can be seen that, if the number of fractions in the chromatography step is reduced to one-third, i.e., 10, the substances that are separated into the fractions S19 to S21 in the case shown in FIG. 4 would be separated into a single fraction, and all molecular groups within about 158 kDa to 20 kDa would be treated as having the same size, thereby making it impossible to obtain accurate size information. Therefore, in the chromatography step, it is necessary that the number of fractions b satisfies at least b ≥ 10, preferably b ≥ 15, and more preferably b ≥ 30.

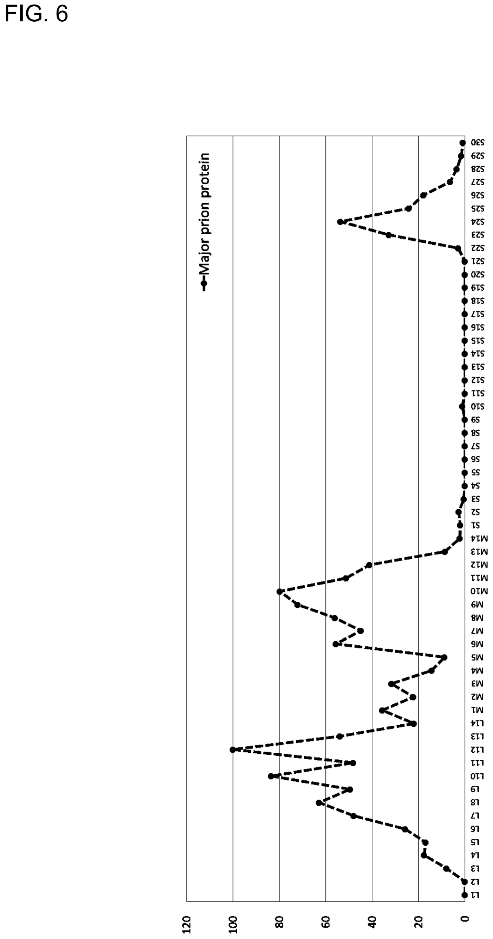

FIG. 6 is a graph showing the results of analysis of the size distribution of the prion proteins in the brain, which is performed based on the fractions L1 to L14, M1 to M14, and S1 to S30 obtained by the procedure described above. In the graph shown in FIG. 6, the horizontal axis represents the fractions L1 to L14, M1 to M14, and S1 to S30 obtained by the biological sample separation and fractionation method according to the present invention, which are arranged in order of recovery. This axis corresponds to an index of the size of the particles contained in each fraction and indicates that the size of the particles decreases from left to right in the figure. The vertical axis represents relative values of signal intensities measured by a mass spectrometer for the respective fractions when a maximum value of a signal intensity measured by the mass spectrometer for a target prion protein is defined as 100. From the graph shown in FIG. 6, it can be seen that the prion proteins present in the brain exist in various sizes and are multimerized, and that quantitative comparison among different sizes is possible. By performing analyses such as those described above, it becomes possible, for example, to distinguish between the brain of a healthy patient and the brain of a patient with Alzheimer's disease or prion disease, which are considered to be caused by the formation of multimers or aggregates, thereby contributing to diagnosis and other applications.

Although the biological sample separation and fractionation method according to the present invention has been described above with reference to the shown embodiments, the present invention is not limited to the described embodiments. For example, in the described embodiments, three stages of centrifugation processes are performed in the stepwise centrifugation step, but two stages or four stages or more centrifugation processes may also be performed. Furthermore, in the density gradient centrifugal recovery steps for the second pellet component and the third pellet component, recovery into 14 fractions is performed, but the number of fractions may be smaller than 14 or greater than 14. Likewise, in the chromatography step, recovery into 30 fractions is performed, but the number of fractions may be smaller or greater than 30.

## Claims

1. A method for separating and fractionating a biological sample, which is collected from a living body, into a plurality of fractions, said method comprising:
a homogenization step of preparing a cell homogenate by adding a solvent to the biological sample and performing cell disruption;
a first centrifugation step of subjecting the prepared cell homogenate to a centrifugation process at a first acceleration G1 to separate the prepared cell homogenate into a first supernatant component and a first pellet component;
a stepwise centrifugation step of performing centrifugation (n-1) times by subjecting a centrifuge tube containing a separated (m-1)-th supernatant component to an m-th centrifugation process at an m-th acceleration Gₘ greater than an (m-1)-th acceleration Gₘ₋₁ to separate the (m-1)-th supernatant component into an m-th supernatant component and an m-th pellet component, where m is an integer of 2 to n, and repeating (n-1) times of separation of the (m-1)-th supernatant component;
a density gradient centrifugal separation step of subjecting, to a density gradient centrifugation process, a predetermined amount of a density gradient sample prepared by adding the m-th pellet component to a density gradient reagent in a density gradient centrifuge tube, said density gradient reagent having a density gradient formed such that a density at a bottom portion of the density gradient centrifuge tube is higher than a density at an upper portion thereof,
a density gradient centrifugal recovery step of recovering, in predetermined quantities according to a predetermined rule, the density gradient sample corresponding to the m-th pellet component in the density gradient centrifuge tube subjected to the density gradient centrifugal separation step, sequentially from an upper side or a bottom side, and fractionating the recovered density gradient sample into aₘ fractions, where aₘ is a predetermined integer corresponding to the m-th pellet component; and
a chromatography step of adding the n-th supernatant component separated by the stepwise centrifugation step to a column of size-exclusion chromatography, subsequently supplying a buffer to the column at a predetermined flow rate to discharge, from the column, a chromatography sample in which the n-th supernatant component and the buffer are mixed, and recovering and fractionating the chromatography sample discharged from the column in predetermined quantities according to a predetermined rule into b fractions, where b is a predetermined integer,
wherein the entire biological sample is fractionated.

2. The method for separating and fractionating a biological sample according to claim 1, wherein, in the density gradient centrifugal recovery step corresponding to the m-th pellet component, the density gradient reagent is evenly fractionated into aₘ fractions in amounts of xₘ ml each, and, in the chromatography step, the chromatography sample discharged from the column is evenly fractionated into b fractions in amounts of y ml each, where xₘ and y are predetermined integers.

3. The method for separating and fractionating a biological sample according to claim 2, wherein xₘ = y.

4. The method for separating and fractionating a biological sample according to claim 3, wherein, when a density gradient centrifuge tube having a capacity of 14 ml is used, xₘ = y = 1 ml, aₘ = 14, and b = 30.

5. The method for separating and fractionating a biological sample according to claim 1, wherein n is 3 and the three centrifugation processes are performed.

6. The method for separating and fractionating a biological sample according to claim 1, wherein the density gradient reagent is an iodixanol solution.

7. The method for separating and fractionating a biological sample according to claim 6, wherein a maximum value of a concentration of the density gradient reagent used when performing the density gradient centrifugal separation step on an (m+1)-th pellet component is determined to be greater than a minimum value of a concentration of the density gradient reagent used when performing the density gradient centrifugal separation step on the m-th pellet component.

8. The method for separating and fractionating a biological sample according to claim 5, wherein the density gradient reagent is an iodixanol solution.

9. The method for separating and fractionating a biological sample according to claim 8, wherein the density gradient reagent is formed to be layered by changing a concentration of the iodixanol solution in six stages.

10. The method for separating and fractionating a biological sample according to claim 9, wherein, in the density gradient reagent used when performing the density gradient centrifugal separation step on a second pellet component, the iodixanol solution is layered such that the concentrations thereof are 35%, 30%, 25%, 22.5%, 20%, and 15%, in order from a lower layer, and, in the density gradient reagent used when performing the density gradient centrifugal separation step on a third pellet component, the iodixanol solution is layered such that the concentrations thereof are 25%, 20%, 15%, 10%, 5%, and 0%, in order from a lower layer.

11. The method for separating and fractionating a biological sample according to claim 1, wherein a plurality of columns connected in series are used in the chromatography step.

12. The method for separating and fractionating a biological sample according to claim 11, wherein two columns connected in series are used in the chromatography step.

13. The method for separating and fractionating a biological sample according to any one of claims 1 to 12, wherein the fractions obtained in each step are automatically separated and recovered by a fraction collector.
